# DEMANDE DE BREVET EUROPEEN

(11) **EP 1 911 438 A2**
(43) Date de publication de la demande: **16.04.2008**
(21) Numéro de dépôt: 07109101.1
(22) Date de dépôt: 29.05.2007
(51) Int. Cl.: A61K 8/35, A61K 8/37, A61K 8/42, A61K 8/49, A61Q 17/04

(54) **Composition cosmétique contenant un dérivé de dibenzoylméthane et un composé amide ou ester d'arylalkyle; procédé de photostabilisation du dérivé de dibenzoylméthane**

(30) Priorité: 23.06.2006 FR 0652621; 23.06.2006 FR 0652620
(71) Demandeur: L'Oréal, 75008 Paris (FR)
(72) Inventeur: Candau, Didier, 91570, BIEVRES (FR); Lucet-Levannier, Karine, 92500, REUIL-MALMAISON (FR)
(74) Mandataire: Miszputen, Laurent

(57) **Abrégé**

La présente invention est relative à une composition cosmétique contenant l'association d'un dérivé du dibenzoylméthane et d'un composé amide ou ester d'arylalkyle particulier de formule (I) ou (II) suivante :

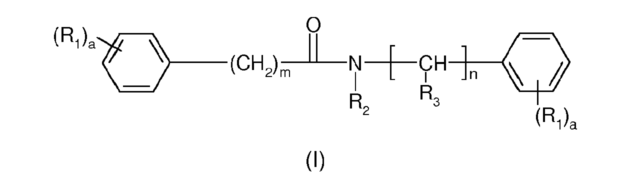

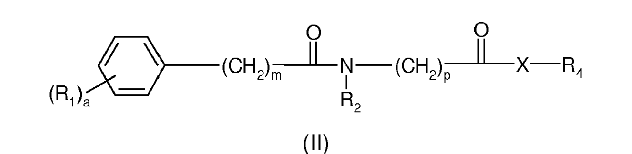

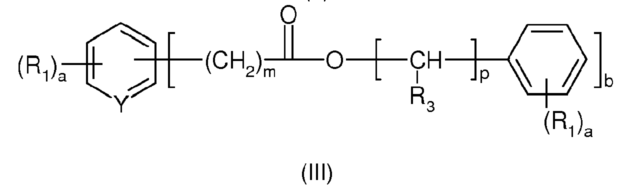

Elle concerne également un procédé de photostabilisation vis-à-vis du rayonnement d'au moins un dérivé du dibenzoylméthane par au moins un composé amide ou ester d'arylalkyle particulier de formule (I), (II) ou (III).

La présente invention est relative également à l'utilisation dudit composé amide ou ester d'arylalkyle particulier de formule (I), (II) ou (III) dans une composition comprenant dans un support cosmétiquement acceptable, au moins un filtre UV du type dérivé du dibenzoylméthane dans le but de d'améliorer l'efficacité de ladite composition vis-à-vis des rayons UV-A.

## Description

La présente invention est relative à une composition cosmétique contenant l'association d'un dérivé du dibenzoylméthane et d'un composé amide ou ester d'arylalkyle particulier de formule (I), (II) ou (III) dont on donnera les définitions ci-après.

Elle concerne également un procédé de photostabilisation vis-à-vis du rayonnement d'au moins un dérivé du dibenzoylméthane par au moins un composé amide ou ester d'arylalkyle particulier de (I), (II) ou (III).

La présente invention est relative également à l'utilisation dudit composé amide ou ester d'arylalkyle particulier de formule (I), (II) ou (III) dans une composition comprenant dans un support cosmétiquement acceptable, au moins un filtre du type dérivé du dibenzoylméthane dans le but de d'améliorer l'efficacité de ladite composition vis-à-vis des rayons UV-A.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.
Dans le but d'assurer une protection de la peau et des matières kératiniques contre le rayonnement UV, on utilise généralement des compositions antisolaires comprenant des filtres organiques, actifs dans l'UV-A et actifs dans l'UV-B. La majorité de ces filtres est liposoluble.

A cet égard, une famille de filtres UV-A particulièrement intéressante est actuellement constituée par les dérivés du dibenzoylméthane, et notamment le 4-ter-butyl-4'-méthoxydibenzoyl méthane, qui présentent en effet un fort pouvoir d'absorption intrinsèque. Ces dérivés du dibenzoylméthane, qui sont maintenant des produits bien connus en soi à titre de filtres actifs dans les UV-A, sont notamment décrits dans les demandes de brevets français FR-A-2326405 et FR-A-2440933, ainsi que dans la demande de brevet européen EP-A-0114607 ; le 4-ter-butyl- 4'-méthoxydibenzoyl méthane est par ailleurs actuellement proposé à la vente sous la dénomination commerciale de « PARSOL 1789 » par la Société DSM NUTRITIONAL PRODUCTS.

Malheureusement, il se trouve que les dérivés du dibenzoylméthane sont des produits relativement sensibles au rayonnement ultraviolet (surtout UV-A), c'est-à-dire, plus précisément, qu'ils présentent une fâcheuse tendance à se dégrader plus ou moins rapidement sous l'action de ce dernier. Ainsi, ce manque substantiel de stabilité photochimique des dérivés du dibenzoylméthane face au rayonnement ultraviolet auquel ils sont par nature destinés à être soumis, ne permet pas de garantir une protection constante durant une exposition solaire prolongée, de sorte que des applications répétées à intervalles de temps réguliers et rapprochés doivent être effectuées par l'utilisateur pour obtenir une protection efficace de la peau contre les rayons UV.

On sait que dans le brevet EP717982 les composés amides ont un effet photostabilisant sur les dérivés de dibenzoylméthane et plus particulièrement les huiles amidées comme le composé N,N-diéthyl-3-méthylbenzamide de structure : 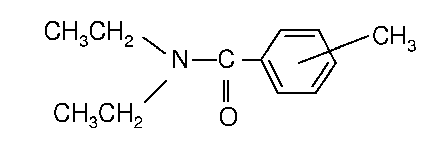 ou le N-butyl, N-acétyl aminopropionate d'éthyle de formule :

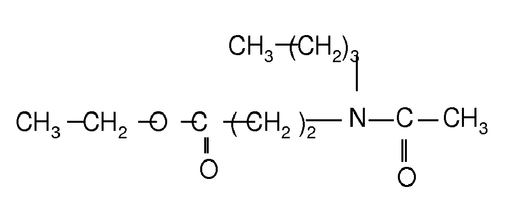

Pour résoudre ce problème, on a déjà proposé dans la demande de brevet EP1618870 d'utiliser des dérivés arylalkyl benzoates en particulier le Benzoate de 2-éthylphenyle comme le produit commercial vendu sous le nom X-TEND 226 ® par la société ISP. Cependant , le niveau de photostabilité du dérivé de dibenzoylméthane après exposition aux UV reste encore insuffisant.

Or, la Demanderesse vient maintenant de découvrir, de façon surprenante, qu'en associant aux dérivés du dibenzoylméthane mentionnés ci-dessus une quantité efficace d'un dérivé amide ou ester d'arylalkyle particulier de formule (I), (II) ou (III) que l'on définira plus loin en détail, il était possible d'améliorer encore de manière substantielle et remarquable, la stabilité photochimique (ou photostabilité) de ces mêmes dérivés du dibenzoylméthane et leur efficacité dans l'UV-A. Les compositions contenant une telle association conduisent également après application à une répartition plus homogène du filtre UV.

Ces découvertes sont à la base de la présente invention.

Ainsi, conformément à l'un des objets de la présente invention, il est maintenant proposé une composition comprenant dans un support cosmétiquement acceptable au moins un système filtrant UV, caractérisée par le fait qu'elle comprend :
(a) au moins un filtre UV-A du type dérivé du dibenzoylméthane et
(b) au moins un composé amide ou ester d'arylalkyle de formule (I), (II) ou (III) dont on donnera les définitions ci-après.

Un autre objet de l'invention concerne également un procédé pour améliorer la stabilité chimique vis-à-vis du rayonnement UV d'au moins un filtre UV-A du type dérivé du dibenzoylméthane consistant à associer audit dérivé de dibenzoylméthane au moins un composé amide dou ester d'arylalkyle particulier de formule (I), (II) ou (III) dont on donnera les définitions ci-après

La présente invention a également enfin pour objet l'utilisation d'un composé amide ou ester d'arylalkyle particulier de formule (I), (II) ou (III), dans une composition comprenant dans un support cosmétiquement acceptable, au moins un dérivé du dibenzoylméthane dans le but de d'améliorer l'efficacité de ladite composition vis-à-vis des rayons UV-A.

D'autres caractéristiques, aspects et avantages de l'invention apparaîtront à la lecture de la description détaillée qui va suivre.

Par « cosmétiquement acceptable », on entend compatible avec la peau et/ou ses phanères, qui présente une couleur, une odeur et un toucher agréables et qui ne génère pas d'inconforts inacceptables (picotements, tiraillements, rougeurs), susceptibles de détourner la consommatrice d'utiliser cette composition.

Parmi les dérivés du dibenzoylméthane, on peut notamment citer, de manière non limitative :
- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5-diméthyldibenzoylméthane,
- le 4,4'-diisopropyldibenzoylméthane,
- le 4,4'-diméthoxydibenzoylméthane,
- le 4-tert.-butyl-4'-méthoxydibenzoylméthane,
- le 2-méthyl-5-isopropyl-4'-méthoxydibenzoylméthane,
- le 2-méthyl-5-tert-butyl-4'-méthxydibenzoylméthane,
- le 2,4-diméthyl-4'-méthoxydibenzoylméthane,
- le 2,6-diméthyl-4-tert-butyl-4'-méthoxydibenzoylméthane.

Parmi les dérivés du dibenzoylméthane mentionnés ci-dessus, on utilisera en particulier le 4-isopropyl-dibenzoylméthane, vendu sous la dénomination de "EUSOLEX 8020" par la Société MERCK, et répondant à la formule suivante :

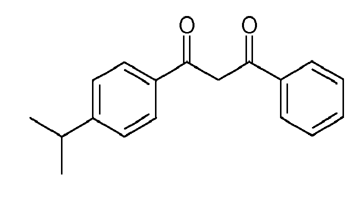

On préfère tout particulièrement mettre en oeuvre le 4-(ter.-butyl) 4'-méthoxy dibenzoylméthane ou Butyl Methoxy Dibenzoylmethane, proposé à la vente sous la dénomination commerciale de "PARSOL 1789" par la Société DSM NUTRITIONAL PRODUCTS ; ce filtre répond à la formule suivante :

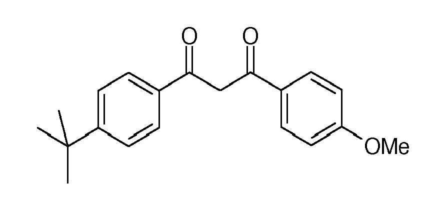

Le ou les dérivés du dibenzoylméthane peuvent être présents dans les compositions conformes à l'invention à des teneurs qui varient de préférence de 0,01 à 10% en poids et plus préférentiellement de 0,1 à 6% en poids par rapport au poids total de la composition.

Les amides ou les esters de d'arylalkyle conformes à l'invention sont choisies par celles répondant aux formules générales (I), (II), (III) suivantes : 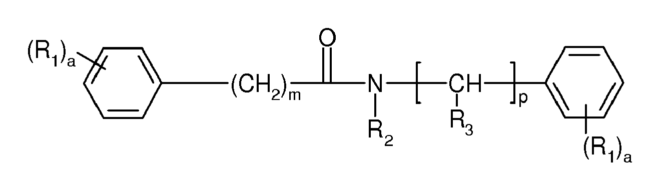 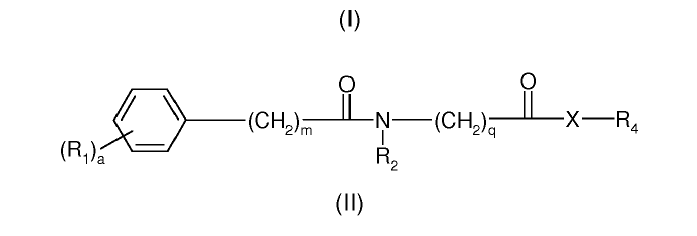 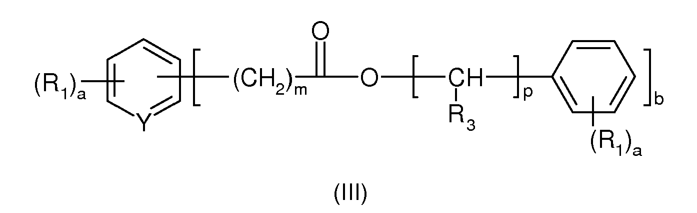 dans lesquelles
- R₁ est un radical alkyle en C₁-C₄, linéaire ou ramifié,
- aest0-2,
- m est 0 - 4,
- b est 1 ou 2,
- R₂ est H, un alkyle en C₁-C₂₀, linéaire ou ramifié, ou le groupement acétyle,
- R₃ de manière indépendante est H ou méthyle,
- p est 0 - 4,
- qest0-3,
- X est O, NH ou NR₅,
- Y est CH ou N,
- R₄ et R₅ sont des radicaux alkyles en C₁-C₂₀, linéaires ou ramifiés,
   avec la condition suivante pour la formule(III) : si Y = CH, R₃ = H, m = 0, p = 2, alors a est différent de 0 et R₁ différent de méthyle.

Dans les formules (I), (II) et (III), parmi les groupes alkyles, on peut notamment citer les groupes méthyle, éthyle, n-propyle, isopropyle, n-butyle, iso-butyle, tert-butyle, éthyle-2 hexyle, dodécyle ou héxadécyle.

Parmi les composés amides de formule (I), on utilisera plus particulièrement les produits (a) à (k) suivants :

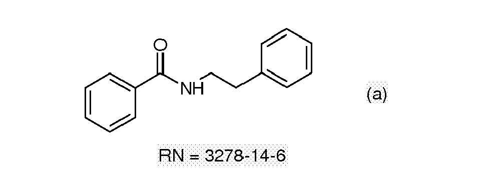

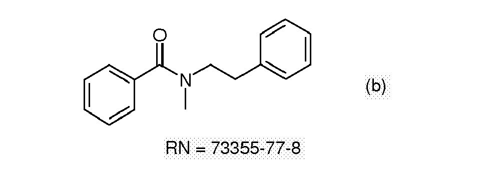

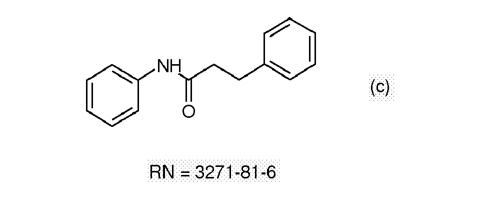

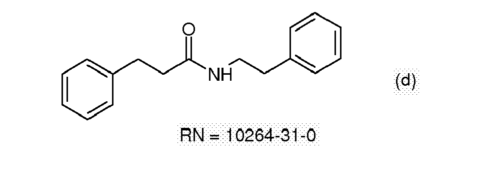

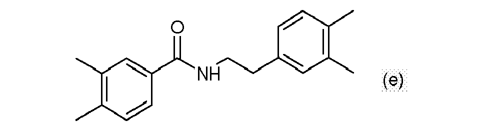

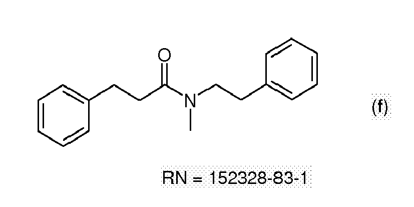

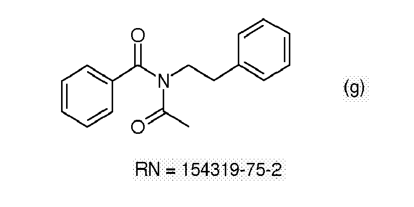

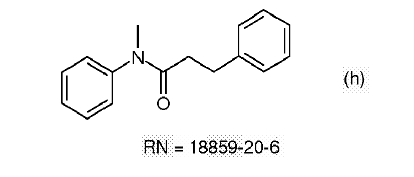

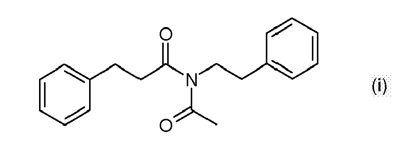

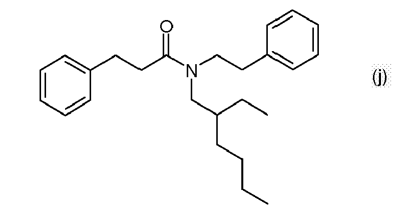

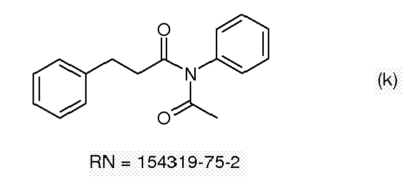

Parmi les composés amides de formule (II), on utilisera plus particulièrement les produits (I) à (r) suivants :

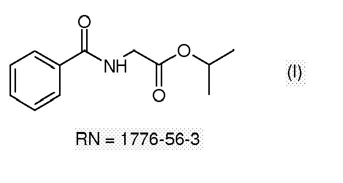

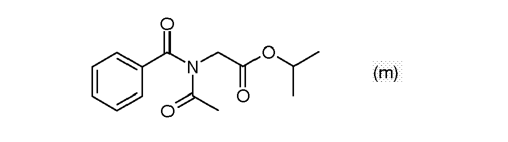

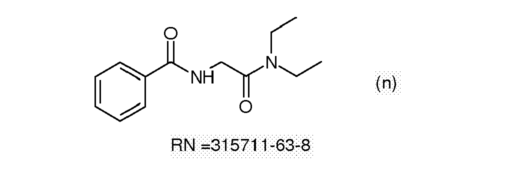

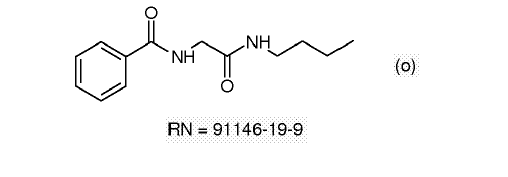

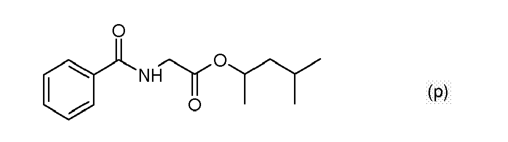

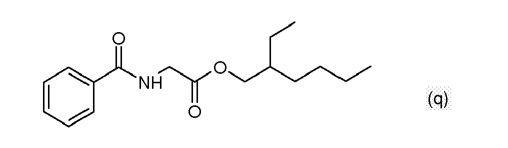

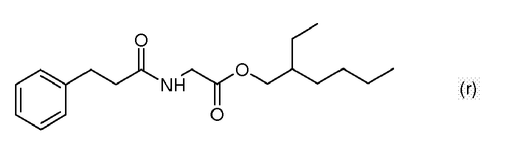

Parmi les composés esters d'arylalkyle de formule (III) mentionnés ci-dessus, on utilisera plus particulièrement les produits (s) à (f) suivants :

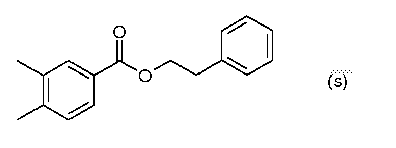

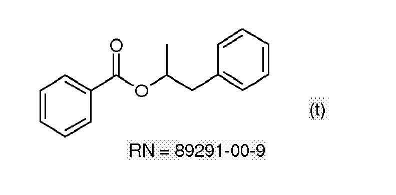

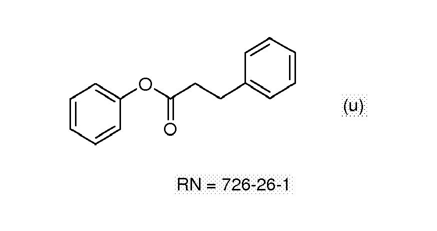

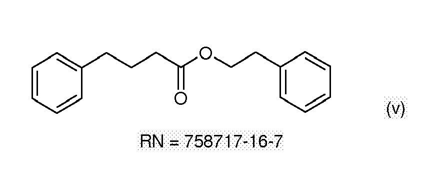

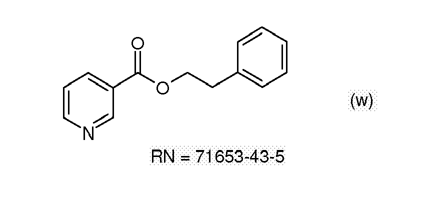

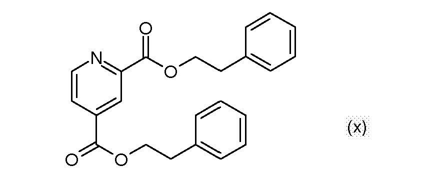

Les composés esters de formules (III) peuvent être préparés par réaction du dérivé acide de formule (2) avec l'alcool de formule (3) en présence d'un catalyseur acide tel par exemple l'acide sulfurique concentré dans un milieu solvant tel par exemple le toluène selon le schéma suivant : 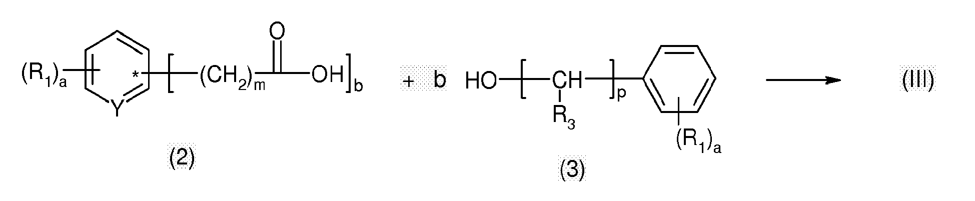 avec les radicaux R₁, R₂, R₃, a, b, m et p ayant la même signification qu'au dessus.

Les composés amides de formule (I) peuvent être préparés par réaction du chlorure d'acide de formule (3) avec l'amine ester de formule (4) en présence d'un capteur d'HCl tel par exemple la triéthylamine dans un milieu solvant tel par exemple l'acétonitrile selon le schéma suivant : 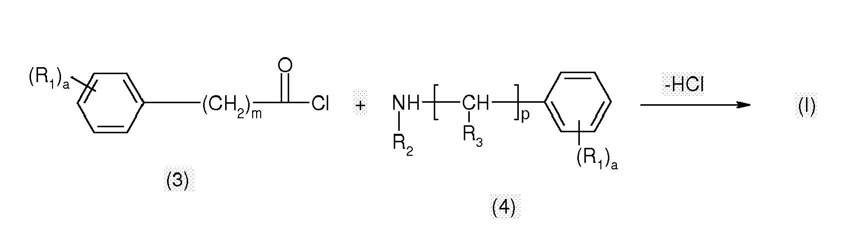 avec les radicaux R₁, R₂, R₃, a, m et p ayant la même signification qu'au dessus.

Les composés amides de formule (II) peuvent être préparés par réaction du dérivé acide de formule (5) avec l'alcool de formule (6) en présence d'un catalyseur acide tel par exemple l'acide sulfurique concentré dans un milieu solvant tel par exemple le toluène selon le schéma suivant :

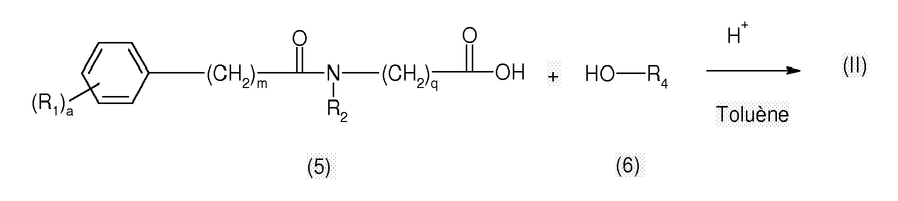

Le ou les composés amides ou esters d'arylalkyle peuvent être présents dans les compositions à des teneurs qui sont généralement comprises entre 0,01 % et 20 % en poids, et de préférence à des teneurs comprises entre 0,1 % et 10 % en poids, par rapport au poids total de la composition.

Selon la présente invention, le (ou les) composé(s) de formules (I), (II) ou (III) sera (seront) utilisé(s) dans une quantité suffisante permettant d'obtenir une amélioration notable et significative de la photostabilité du dérivé du dibenzoylméthane dans une composition donnée. Cette quantité minimale en agent photostabilisant à mettre en oeuvre peut varier selon la quantité de dibenzoylméthane présent au départ dans la composition et selon la nature du support cosmétiquement acceptable retenu pour la composition. Elle peut être déterminée sans aucune difficulté au moyen d'un test classique de mesure de photostabilité.

Les compositions conformes à l'invention peuvent comporter en plus d'autres filtres UV organiques ou inorganiques complémentaires actifs dans l'UVA et/ou l'UVB hydrosolubles ou liposolubles ou bien insolubles dans les solvants cosmétiques couramment utilisés.

Les filtres UV organiques complémentaires sont notamment choisis parmi les anthranilates ; les dérivés cinnamiques ; les dérivés salicyliques, les dérivés du camphre ; les dérivés de la benzophénone ; les dérivés de β,β-diphénylacrylate ; les dérivés de triazine ; les dérivés de benzotriazole ; les dérivés de benzalmalonate notamment ceux cités dans le brevet US5624663 ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US 5,166,355, GB2303549, DE 197 26 184 et EP893119 ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Comme exemples de filtres UV organiques complémentaires, on peut citer ceux désignés ci-dessous sous leur nom INCI :

### Dérivés de l'acide para-aminobenzoique :

PABA,

Ethyl PABA,

Ethyl Dihydroxypropyl PABA,

Ethylhexyl Diméthyl PABA vendu notamment sous le nom « ESCALOL 507 » par ISP, Glyceryl PABA,

PEG-25 PABA vendu sous le nom « UVINUL P25 » par BASF,

### Dérivés salicyliques :

Homosalate vendu sous le nom « Eusolex HMS » par Rona/EM Industries,

Ethylhexyl Salicylate vendu sous le nom « NEO HELIOPAN OS » par Haarmann et REIMER,

Dipropyleneglycol Salicylate vendu sous le nom « DIPSAL » par SCHER,

TEA Salicylate, vendu sous le nom « NEO HELIOPAN TS » par Haarmann et REIMER,

### Dérivés cinnamiques :

Ethylhexyl Methoxycinnamate vendu notamment sous le nom commercial « PARSOL MCX » par HOFFMANN LA ROCHE,

Isopropyl Methoxy cinnamate,

Isoamyl Methoxy cinnamate vendu sous le nom commercial « NEO HELIOPAN E 1000 » par HAARMANN et REIMER,

Cinoxate,
DEA Methoxycinnamate,
- Diisopropyl Methylcinnamate,
   Glyceryl Ethylhexanoate Dimethoxycinnamate

### Dérivés de β,β-diphénylacrylate :

Octocrylene vendu notamment sous le nom commercial « UVINUL N539 » par BASF, Etocrylene, vendu notamment sous le nom commercial « UVINUL N35 » par BASF,

### Dérivés de la benzophénone :

Benzophenone-1 vendu sous le nom commercial « UVINUL 400 » par BASF,

Benzophenone-2 vendu sous le nom commercial « UVINUL D50 » par BASF

Benzophenone-3 ou Oxybenzone, vendu sous le nom commercial « UVINUL M40 » par BASF,

Benzophenone-4 vendu sous le nom commercial « UVINUL MS40 » par BASF, Benzophenone-5

Benzophenone-6 vendu sous le nom commercial « Helisorb 11 » par Norquay

Benzophenone-8 vendu sous le nom commercial « Spectra-Sorb UV-24 » par American Cyanamid

Benzophenone-9 vendu sous le nom commercial« UVINUL DS-49» par BASF, Benzophenone-12

2-(4-diethylamino-2-hydroxybenzoyl)-benzoate de n-hexyle.

### Dérivés du benzylidène camphre :

3-Benzylidene camphor fabriqué sous le nom « MEXORYL SD» par CHIMEX,

4-Methylbenzylidene camphor vendu sous le nom « EUSOLEX 6300 » par MERCK ,

Benzylidene Camphor Sulfonic Acid fabriqué sous le nom « MEXORYL SL» par CHIMEX,

Camphor Benzalkonium Methosulfate fabriqué sous le nom « MEXORYL SO » par CHIMEX,

Terephthalylidene Dicamphor Sulfonic Acid fabriqué sous le nom « MEXORYL SX » par CHIMEX,

Polyacrylamidomethyl Benzylidene Camphor fabriqué sous le nom « MEXORYL SW » par CHIMEX,

### Dérivés du phenyl benzimidazole :

Phenylbenzimidazole Sulfonic Acid vendu notamment sous le nom commercial « EUSOLEX 232 » par MERCK,

Disodium Phenyl Dibenzimidazole Tetra-sulfonate vendu sous le nom commercial commercial « NEO HELIOPAN AP » par Haarmann et REIMER,

### Dérivés du phenyl benzotriazole :

Drometrizole Trisiloxane vendu sous le nom « Silatrizole » par RHODIA CHIMIE ,

Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

### Dérivés de triazine :

Bis-Ethylhexyloxyphenol Methoxyphenyl Triazine vendu sous le nom commercial «TINOSORB S » par CIBA GEIGY,

Ethylhexyl triazone vendu notamment sous le nom commercial «UVINUL T150 » par BASF,

Diethylhexyl Butamido Triazone vendu sous le nom commercial « UVASORB HEB » par SIGMA 3V,

2,4,6-tris(4'-amino benzalmalonate de dinéopentyle)-s-triazine

la 2,4,6-tris-(4'-amino benzalmalonate de diisobutyle)-s-triazine.

les filtres triazines symétriques décrits dans le brevet US6,225,467, la demande WO2004/085412 (voir composés 6 et 9) ou le document «Symetrical Triazine Derivatives» IP.COM Journal , IP.COM INC WEST HENRIETTA, NY, US (20 septembre 2004) notamment les 2,4,6-tris-(biphényl)-1,3,5-triazines (en particulier la 2,4,6-tris(biphenyl-4-yl-1,3,5-triazine) et la 2,4,6-tris(terphenyl)-1,3,5-triazine qui sont reprises dans les demandes de brevet WO06/035000, WO06/034982, WO06/034991, WO06/035007, WO2006/034992, W02006/034985.

### Dérivés anthraniliques :

Menthyl anthranilate vendu sous le nom commercial commercial « NEO HELIOPAN MA » par Haarmann et REIMER,

### Dérivés d'imidazolines :

Ethylhexyl Dimethoxybenzylidene Dioxoimidazoline Propionate,

### Dérivés du benzalmalonate :

Di-néopentyl 4'-méthoxybenzalmalonate

Polyorganosiloxane à fonctions benzalmalonate comme le Polysilicone-15 vendu sous la dénomination commerciale « PARSOL SLX » par HOFFMANN LA ROCHE

### Dérivés de 4,4-diarylbutadiène :

- 1,1-dicarboxy (2,2'-diméthyl-propyl)-4,4-diphénylbutadiène

### Dérivés de benzoxazole :

2,4-bis-[5-1 (diméthylpropyl)benzoxazol-2-yl-(4-phenyl)-imino]-6-(2-ethylhexyl)-imino-1,3,5-triazine vendu sous le nom d'Uvasorb K2A par Sigma 3V
et leurs mélanges.

Les filtres UV organiques complémentaires préférentiels sont choisis parmi
Ethylhexyl Methoxycinnamate
Homosalate
Ethylhexyl Salicylate,
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
Methylène bis-Benzotriazolyl Tetramethylbutylphénol
Ethylhexyl triazone,
Bis-Ethylhexyloxyphenol Methoxyphenyl Triazine
Diethylhexyl Butamido Triazone,
2,4,6-tris(4'-amino benzalmalonate de dinéopentyle)-s-triazine
la 2,4,6-tris-(4'-amino benzalmalonate de diisobutyle)-s-triazine.
la 2,4,6-tris(biphenyl-4-yl-1,3,5-triazine)
la 2,4,6-tris(terphenyl)-1,3,5-triazine
Drometrizole Trisiloxane
Polysilicone-15
Di-néopentyl 4'-méthoxybenzalmalonate
1,1-dicarboxy (2,2'-diméthyl-propyl)-4,4-diphénylbutadiène
2,4-bis-[5-1(diméthylpropyl)benzoxazol-2-yl-(4-phenyl)-imino]-6-(2-ethylhexyl)-imino-1,3,5-triazine
et leurs mélanges.

Les filtres UV inorganiques sont choisis parmi des pigments d'oxydes métalliques enrobés ou non ayant une taille moyenne des particules primaires: généralement entre 5 nm et 100 nm, de préférence entre 10 nm et 50 nm comme par exemple des pigments d'oxyde de titane (amorphe ou cristallisé sous forme rutile et/ou anatase), de fer, de zinc, de zirconium ou de cérium qui sont tous des agents photoprotecteurs UV bien connus en soi.

Les pigments peuvent être enrobés ou non enrobés.

Les pigments enrobés sont des pigments qui ont subi un ou plusieurs traitements de surface de nature chimique, électronique, mécanochimique et/ou mécanique avec des composés tels que décrits par exemple dans Cosmetics & Toiletries, Février 1990, Vol. 105, p. 53-64, tels que des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), du polyéthylène, des silicones, des protéines (collagène, élastine), des alcanolamines, des oxydes de silicium, des oxydes métalliques ou de l'hexamétaphosphate de sodium.

De façon connue, les silicones sont des polymères ou oligomères organo-siliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane), des radicaux hydrocarbonés éventuellement substitués étant directement liés par l'intermédiaire d'un atome de carbone sur lesdits atomes de silicium.

Le terme "silicones" englobe également les silanes nécessaires à leur préparation, en particulier, les alkyl silanes.

Les silicones utilisées pour l'enrobage des pigments convenant à la présente invention sont de préférence choisies dans le groupe contenant les alkyl silanes, les polydialkylsiloxanes, et les polyalkylhydrogénosiloxanes. Plus préférentiellement encore, les silicones sont choisies dans le groupe contenant l'octyl triméthyl silane, les polydiméthylsiloxanes et les polyméthylhydro-génosiloxanes.

Bien entendu, les pigments d'oxydes métalliques avant leur traitement par des silicones, peuvent avoir été traités par d'autres agents de surface, en particulier par de l'oxyde de cérium, de l'alumine, de la silice, des composés de l'aluminium, des composés du silicium, ou leurs mélanges.

Les pigments enrobés sont plus particulièrement des oxydes de titane enrobés :
- de silice tels que le produit "SUNVEIL" de la société IKEDA et le produit "Eusolex T-AVO" de la société MERCK
- de silice et d'oxyde de fer tels que le produit "SUNVEIL F" de la société IKEDA,
- de silice et d'alumine tels que les produits "MICROTITANIUM DIOXIDE MT 500 SA" et_"MICROTITANIUM DIOXIDE MT 100 SA" de la société TAYCA, "TIOVEIL" de la société TIOXIDE, et « Mirasun TiW 60 » de la société Rhodia,
- d'alumine tels que les produits "TIPAQUE TTO-55 (B)" et "TIPAQUE TTO-55 (A)" de la société ISHIHARA, et "UVT 14/4" de la société KEMIRA,
- d'alumine et de stéarate d'aluminium tels que le produit "MICROTITANIUM DIOXIDE MT 100 TV, MT 100 TX, MT 100 Z, MT-01 de la société TAYCA, les produits "Solaveil CT-1 0 W", "Solaveil CT 100" et "Solaveil CT 200" de la société UNIQEMA,
- de silice, d'alumine et d'acide alginique tel que le produit " MT-100 AQ" de la société TAYCA,
- d'alumine et de laurate d'aluminium tel que le produit "MICROTITANIUM DIOXIDE MT 100 S" de la société TAYCA,
- d'oxyde de fer et de stéarate de fer tels que le produit "MICROTITANIUM DIOXIDE MT 100 F" de la société TAYCA,
- d'oxyde de zinc et de stéarate de zinc tels que le produit "BR351" de la société TAYCA,
- de silice et d'alumine et traités par une silicone tels que les produits "MICROTITANIUM DIOXIDE MT 600 SAS", "MICROTITANIUM DIOXIDE MT 500 SAS" ou "MICROTITANIUM DIOXIDE MT 100 SAS"de la société TAYCA,
- de silice, d'alumine, de stéarate d'aluminium et traités par une silicone tels que le produit "STT-30-DS" de la société TITAN KOGYO,
- de silice et traité par une silicone tel que le produit "UV-TITAN X 195" de la société KEMIRA, ou le produit SMT-100 WRS de la société TAYCA.
- d'alumine et traités par une silicone tels que les produits "TIPAQUE TTO-55 (S)" de la société ISHIHARA, ou "UV TITAN M 262" de la société KEMIRA, de triéthanolamine tels que le produit "STT-65-S" de la société TITAN KOGYO,
- d'acide stéarique tels que le produit "TIPAQUE TTO-55 (C)" de la société ISHIHARA,
- d'hexamétaphosphate de sodium tels que le produit "MICROTITANIUM DIOXIDE MT 150 W" de la société TAYCA.
   D'autres pigments d'oxyde de titane traités avec une silicone sont de préférence le TiO₂ traité par l'octyl triméthyl silane et dont la taille moyenne des particules élémentaires est comprise entre 25 et 40 nm tel que celui vendu sous la dénomination commerciale "T 805" par la société DEGUSSA SILICES, le TiO2 traité par un polydiméthylsiloxane et dont la taille moyenne des particules élémentaires est de 21 nm tel que celui vendu sous la dénomination commerciale "70250 Cardre UF TiO2SI3" par la société CARDRE, le TiO₂ anatase/rutile traité par un polydiméthylhydrogénosiloxane et dont la taille moyenne des particules élémentaires est de 25 nm tel que celui vendu sous la dénomination commerciale "MICRO TITANIUM DIOXYDE USP GRADE HYDROPHOBIC" par la société COLOR TECHNIQUES.
   Les pigments d'oxyde de titane non enrobés sont par exemple vendus par la société TAYCA sous les dénominations commerciales "MICROTITANIUM DIOXIDE MT 500 B" ou "MICROTITANIUM DIOXIDE MT600 B", par la société DEGUSSA sous la dénomination "P 25", par la société WACKHER sous la dénomination "Oxyde de titane transparent PW", par la société MIYOSHI KASEI sous la dénomination "UFTR", par la société TOMEN sous la dénomination "ITS" et par la société TIOXIDE sous la dénomination "TIOVEIL AQ".
   Les pigments d'oxyde de zinc non enrobés, sont par exemple
- ceux commercialisés sous la dénomination "Z-cote" par la société Sunsmart ;
- ceux commercialisés sous la dénomination "Nanox" par la société Elementis ;
- ceux commercialisés sous la dénomination "Nanogard WCD 2025" par la société Nanophase Technologies ;

Les pigments d'oxyde de zinc enrobés sont par exemple
- ceux commercialisés sous la dénomination « Z-COTE HP1 » par la société SUNSMART (ZnO enrobé dimethicone) ;
- ceux commercialisés sous la dénomination "Oxide zinc CS-5" par la société Toshibi (ZnO enrobé par polymethylhydrogenesiloxane) ;
- ceux commercialisés sous la dénomination "Nanogard Zinc Oxide FN" par la société Nanophase Technologies (en dispersion à 40% dans le Finsolv TN, benzoate d'alcools en C12-C15) ;
- ceux commercialisés sous la dénomination "DAITOPERSION ZN-30" et "DAITOPERSION Zn-50" par la société Daito (dispersions dans cyclopolyméthylsiloxane/polydiméthylsiloxane oxyéthyléné, contenant 30% ou 50% de nano-oxydes de zinc enrobés par la silice et le polyméthylhydrogènesiloxane) ;
- ceux commercialisés sous la dénomination "NFD Ultrafine ZnO" par la société Daikin (ZnO enrobé par phosphate de perfluoroalkyle et copolymère à base de perfluoroalkyléthyle en dispersion dans du cyclopentasiloxane) ;
- ceux commercialisés sous la dénomination "SPD-Z1" par la société Shin-Etsu (ZnO enrobé par polymère acrylique greffé silicone, dispersé dans cyclodiméthylsiloxane) ;
- ceux commercialisés sous la dénomination "Escalol Z100" par la société ISP (ZnO traité alumine et dispersé dans le mélange methoxycinnamate d'ethylhexyle /copolymère PVP-hexadecene / methicone) ;
- ceux commercialisés sous la dénomination "Fuji ZnO-SMS-10" par la société Fuji Pigment (ZnO enrobé silice et polymethylsilsesquioxane) ;
- ceux commercialisés sous la dénomination "Nanox Gel TN" par la société Elementis (ZnO dispersé à 55% dans du benzoate d'alcools en C12-C15 avec polycondensat d'acide hydroxystéarique).

Les pigments d'oxyde de cérium non enrobé sont vendus par exemple sous la dénomination "COLLOIDAL CERIUM OXIDE" par la société RHONE POULENC.
Les pigments d'oxyde de fer non enrobés sont par exemple vendus par la société ARNAUD sous les dénominations "NANOGARD WCD 2002 (FE 45B)", "NANOGARD IRON FE 45 BL AQ", "NANOGARD FE 45R AQ, "NANOGARD WCD 2006 (FE 45R)", ou par la société MITSUBISHI sous la dénomination "TY-220".

Les pigments d'oxyde de fer enrobés sont par exemple vendus par la société ARNAUD sous les dénominations "NANOGARD WCD 2008 (FE 45B FN)", "NANOGARD WCD 2009 (FE 45B 556)", "NANOGARD FE 45 BL 345", "NANOGARD FE 45 BL", ou par la société BASF sous la dénomination "OXYDE DE FER TRANSPARENT".

On peut également citer les mélanges d'oxydes métalliques, notamment de dioxyde de titane et de dioxyde de cérium, dont le mélange équipondéral de dioxyde de titane et de dioxyde de cérium enrobés de silice, vendu par la société IKEDA sous la dénomination "SUNVEIL A", ainsi que le mélange de dioxyde de titane et de dioxyde de zinc enrobé d'alumine, de silice et de silicone tel que le produit "M 261 " vendu par la société KEMIRA ou enrobé d'alumine, de silice et de glycérine tel que le produit "M 211 " vendu par la société KEMIRA.

Les agents photoprotecteurs additionnels sont généralement présents dans les compositions selon l'invention dans des proportions allant de 0,01 à 20% en poids par rapport au poids total de la composition, et de préférence allant de 0,1 à 10% en poids par rapport au poids total de la composition.

Les compositions selon l'invention peuvent également contenir des agents de bronzage et/ou de brunissage artificiels de la peau (agents autobronzants), et plus particulièrement la dihydroxyacétone (DHA). Ils sont présents de préférence dans des quantité allant 0,1 à 10% en poids par rapport au poids total de la composition.

Les compositions aqueuses conformes à la présente invention peuvent comprendre en outre des adjuvants cosmétiques classiques notamment choisis parmi les corps gras, les solvants organiques, les épaississants ioniques ou non ioniques, hydrophiles ou lipophiles, les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, des actifs, les charges, les polymères, les propulseurs, les agents alcalinisants ou acidifiants ou tout autre ingrédient habituellement utilisé dans le domaine cosmétique et/ou dermatologique.

Les corps gras peuvent être constitués par une huile ou une cire autre que les cires apolaires telles que définies précédemment ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine); végétales (huile d'amande douce, de macadamia, de pépin de cassis, de jojoba) ; synthétiques comme le perhydrosqualène, les alcools gras, les amides grasses (comme l'isopropyl lauroyl sarcosinate vendu sous la dénomination d' « Eldew SL-205 » par la société Ajinomoto), les acides ou les esters gras (comme le benzoate d'alcools en C12-C15 vendu sous la dénomination commerciale « Finsolv TN » ou « Witconol TN » par la société WITCO, le palmitate d'octyle, le lanolate d'isopropyle, les triglycérides dont ceux des acides caprique/caprylique, le dicaprylyl carbonate vendu sous la dénomination « Cetiol CC » par la société Cognis), les esters et éthers gras oxyéthylénés ou oxypropylénés; les huiles siliconées (cyclométhicone, polydiméthysiloxanes ou PDMS) ou fluorées, les polyalkylènes.

Comme composés cireux, on peut citer la cire de carnauba, la cire d'abeille, l'huile de ricin hydrogénée, les cires de polyéthylène et les cires de polyméthylène comme celle vendue sous la dénomination Cirebelle 303 par la société SASOL.

Parmi les solvants organiques, on peut citer les alcools et polyols inférieurs. Ces derniers peuvent être choisis parmi les glycols et les éthers de glycol comme l'éthylène glycol, le propylène glycol, le butylène glycol, le dipropylène glycol ou le diéthylène glycol.

Comme épaississants hydrophiles, on peut citer les polymères carboxyvinyliques tels que les Carbopols (Carbomers) et les Pemulen (Copolymère acrylate/C10-C30-alkylacrylate) ; les polyacrylamides comme par exemple les copolymères réticulés vendus sous les noms Sepigel 305 (nom C.T.F.A. : polyacrylamide/C13-14 isoparaffin/Laureth 7) ou Simulgel 600 (nom C.T.F.A. : acrylamide / sodium acryloyldimethyltaurate copolymer / isohexadecane / polysorbate 80) par la société Seppic ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Hoechst sous la dénomination commerciale « Hostacerin AMPS » (nom CTFA : ammonium polyacryloyldimethyl taurate ou le SIMULGEL 800 commercialisé par la société SEPPIC (nom CTFA : sodium polyacryolyldimethyl taurate / polysorbate 80 / sorbitan oleate) ; les copolymères d'acide 2-acrylamido 2-méthylpropane sulfonique et d'hydroxyethyl acrylate comme le SIMULGEL NS et le SEPINOV EMT 10 commercialisés par la société SEPPIC ; les dérivés cellulosiques tels que l'hydroxyéthylcellulose ; les polysaccharides et notamment les gommes telles que la gomme de Xanthane ; et leurs mélanges.

Comme épaississants lipophiles, on peut citer les polymères synthétiques tels que les poly C10-C30 alkyl acrylates vendu sous la dénomination « INTELIMER IPA 13-1 » et « INTELIMER IPA 13-6 » par la société Landec ou encore les argiles modifiées telles que l'hectorite et ses dérivés, comme les produits commercialisés sous les noms de Bentone.

Parmi les actifs, on peut citer :
- les vitamines (A, C, E, K, PP...) et leurs dérivés ou précurseurs, seuls ou en mélanges,
- les agents anti-pollution et/ou agent anti-radicalaire ;
- les agents dépigmentants et/ou des agents pro-pigmentants ;
- les agents anti-glycation ;
- les agents apaisants,
- les inhibiteurs de NO-synthase ;
- les agents stimulant la synthèse de macromolécules dermiques ou épidermiques et/ou empêchant leur dégradation ;
- les agents stimulant la prolifération des fibroblastes ;
- les agents stimulant la prolifération des kératinocytes ;
- les agents myorelaxants;
- les agents tenseurs,
- les agents matifiants,
- les agents kératolytiques,
- les agents desquamants ;
- les agents hydratants ;
- les agents anti-inflammatoires ;
- les agents agissant sur le métabolisme énergétique des cellules,
- les agents répulsifs contre les insectes
- les antagonistes de substances P ou de CRGP.
- les agents anti-chute et/ou repousse des cheveux
- les agents anti-rides.

Bien entendu, l'homme de l'art veillera à choisir le ou les éventuels composés complémentaires cités ci-dessus et/ou leurs quantités de manière telle que les propriétés avantageuses attachées intrinsèquement aux compositions conformes à l'invention ne soient pas, ou substantiellement pas, altérées par la ou les adjonctions envisagées.

Les compositions selon l'invention peuvent être préparées selon les techniques bien connues de l'homme de l'art. Elles peuvent se présenter en particulier sous forme d'émulsion, simple ou complexe (H/E, E/H, H/E/H ou E/H/E) telle qu'une crème, un lait ou d'un gel crème ; sous la forme d'un gel aqueux ; sous la forme d'une lotion. Elles peuvent éventuellement être conditionnées en aérosol et se présenter sous forme de mousse ou de spray.

De préférence, les compositions selon l'invention se présentent sous la forme d'une émulsion huile-dans-eau ou eau-dans huile.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E). Les émulsions peuvent contenir également d'autres types de stabilisants comme par exemple des charges, des polymères gélifiants ou épaississants.

Comme tensioactifs émulsionnants utilisables pour la préparation des émulsions E/H, on peut citer par exemple les alkyl esters ou éthers de sorbitane, de glycérol ou de sucres ; les tensioactifs siliconés comme les diméthicone copolyols tels que le mélange de cyclométhicone et de diméthicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning ; le Cetyl dimethicone copolyol tel que le produit vendu sous la dénomination Abil EM 90R par la société Goldschmidt et le mélange de cétyl diméthicone copolyol, d'isostéarate de polyglycérole (4 moles) et de laurate d'hexyle vendu sous la dénomination ABIL WE 09 par la société Goldschmidt. On peut y ajouter aussi un ou plusieurs co-émulsionnants, qui, de manière avantageuse, peuvent être choisis dans le groupe comprenant les esters alkylés de polyol.

Comme esters alkylés de polyol, on peut citer notamment les esters de polyéthylèneglycol comme le PEG-30 Dipolyhydroxystearate tel que le produit commercialisé sous le nom Arlacel P135 par la socité ICI ;

Comme esters de glycérol et/ou de sorbitan, on peut citer par exemple l'isostéarate de polyglycérol, tel que le produit commercialisé sous la dénomination Isolan GI 34 par la société Goldschmidt ; l'isostéarate de sorbitan, tel que le produit commercialisé sous la dénomination Arlacel 987 par la société ICI ; l'isostéarate de sorbitan et le glycérol, tel que le produit commercialisé sous la dénomination Arlacel 986 par la société ICI, et leurs mélanges.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) comme le mélange PEG-100 Stearate/ Glyceryl Stearate commercialisé par exemple par la société ICI sous la dénomination Arlacel 165 ; les éthers d'alcools gras oxyalkylénés (oxyéthylénés et/ou oxypropylénés) ; les esters de sucres comme le stéarate de sucrose ; les éthers d'alcool gras et de sucre, notamment les alkylpolyglucosides (APG) tels que le décylglucoside et le laurylglucoside commercialisés par exemple par la société Henkel sous les dénominations respectives Plantaren 2000 et Plantaren 1200, le cétostéarylglucoside éventuellement en mélange avec l'alcool cétostéarylique, commercialisé par exemple sous la dénomination Montanov 68 par la société Seppic, sous la dénomination Tegocare CG90 par la société Goldschmidt et sous la dénomination Emulgade KE3302 par la société Henkel, ainsi que l'arachidyl glucoside, par exemple sous la forme du mélange d'alcools arachidique et béhénique et d'arachidylglucoside commercialisé sous la dénomination Montanov 202 par la société Seppic. Selon un mode particulier de réalisation de l'invention, le mélange de l'alkylpolyglucoside tel que défini ci-dessus avec l'alcool gras correspondant peut être sous forme d'une composition auto-émulsionnante, comme décrit par exemple dans le document WO-A-92/06778.

Parmi les autres stabilisants d'émulsion, on utilisera plus particulièrement les polymères d'acide isophtalique ou d'acide sulfoisophtalique, et en particulier les copolymères de phtalate / sulfoisophtalate / glycol par exemple le copolymère de Diéthylèneglycol / Phtalate / Isophtalate / 1,4-cyclohexane-diméthanol (nom INCI : Polyester-5) vendu sous les dénominations "Eastman AQ polymer" (AQ35S, AQ38S, AQ55S, AQ48 Ultra) par la société Eastman Chemical.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Les compositions selon l'invention trouvent leur application dans un grand nombre de traitements, notamment cosmétiques, de la peau, des lèvres et des cheveux, y compris le cuir chevelu, notamment pour la protection et/ou le soin de la peau, des lèvres et/ou des cheveux, et/ou pour le maquillage de la peau et/ou des lèvres.

Un autre objet de la présente invention est constitué par l'utilisation des compositions selon l'invention telles que ci-dessus définies pour la fabrication de produits pour le traitement cosmétique de la peau, des lèvres, des ongles, des cheveux, des cils, sourcils et/ou du cuir chevelu, notamment des produits de soin, des produits de protection solaire et des produits de maquillage.

Les compositions cosmétiques selon l'invention peuvent par exemple être utilisées comme produit de maquillage.

Les compositions cosmétiques selon l'invention peuvent par exemple être utilisées comme produit de soin et/ou de protection solaire pour le visage et/ou le corps de consistance liquide à semi-liquide, telles que des laits, des crèmes plus ou moins onctueuses, gel-crèmes, des pâtes. Elles peuvent éventuellement être conditionnées en aérosol et se présenter sous forme de mousse ou de spray.

Les compositions selon l'invention sous forme de lotions fluides vaporisables conformes à l'invention sont appliquées sur la peau ou les cheveux sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517 (faisant partie intégrante du contenu de la description).

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane. Ils sont présents de préférence dans des quantités allant de 15 à 50% en poids par rapport au poids total de la composition.

Des exemples concrets, mais nullement limitatifs, illustrant l'invention, vont maintenant être donnés.

### EXEMPLES DE SYNTHESE

### EXEMPLE 1 : Préparation du 1,3-dimethylbutyl (benzoylamino)acetate :

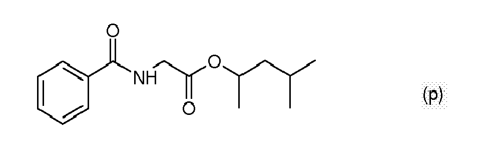

On chauffe à 80°C pendant 17 heures un mélange d'acide hippurique (8 g, 0,0446 mole), de 4-méthyl-2- pentanol (6,8 ml, 0,0536 mole) et d'acide sulfurique à 98% (0,41 ml, 0,00446 mole) dans 100 ml de toluène. Un deuxième ajout de 4-méthyl-2-pentanol (6,8 ml, 0,0536 mole) et d'acide sulfurique à 98% (0,41 ml, 0,00446 mole) est effectué et le mélange réactionnel est porté au reflux pendant 7 heures. Le mélange réactionnel est évaporé à sec sous pression réduite. Le résidu est repris dans l'acétate d'éthyle et lavé deux fois à l'eau. La phase organique isolée est séchée sur sulfate de sodium. Après filtration et évaporation du solvant, l'huile jaune obtenue est chromatographiée sur Silice (éluant : Heptane/EtOAc 80 :20), pour donner 3 g (Rendement : 22%) de fractions propres du composé de l'exemple 1 sous forme d'une huile jaune pâle.

### EXEMPLE 2 : Préparation du 2-ethylhexyl (benzoylamino)acetate :

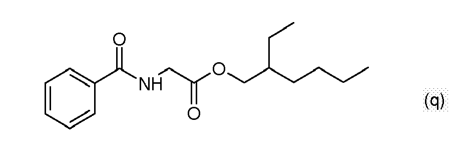

On chauffe au reflux pendant 17 heures un mélange d'acide hippurique (6,25 g, 0,0349 mole), de 2-éthyl-1-hexanol (6 ml, 0,0384 mole) et d'acide sulfurique à 98% (0,18 ml, 0,00349 mole) dans 20 ml de toluène. Le mélange réactionnel est évaporé à sec sous pression réduite. Le résidu est repris dans l'acétate d'éthyle et lavé deux fois à l'eau. La phase organique isolée est séchée sur sulfate de sodium. Après filtration et évaporation du solvant, l'huile jaune obtenue est chromatographiée sur Silice (éluant : CH2Cl2), pour donner 8,8 g (Rendement : 87%) de fractions propres du composé de l'exemple 2 sous forme d'une huile jaune pâle.

### EXEMPLE 3 : Préparation du 2-ethylhexyl [(3-phenylpropanoyl)amino]acetate :

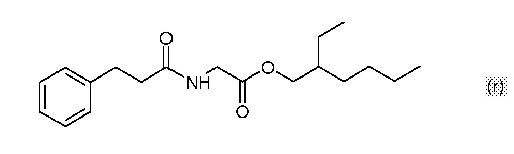

### Première étape : Préparation du 2-ethylhexyl aminoacetate :

On chauffe au reflux pendant 17 heures un mélange de glycine (4,36 g, 0,0582 mole), de 2-éthyl-1-hexanol (10 ml, 0,0639 mole) et d'acide sulfurique à 98% (0,311 ml, 0,00581 mole) dans 20 ml de toluène. On ajoute 50 ml de toluène à la suspension obtenue et ajoute 0,5 ml d'acide sulfurique concentré et porte le tout au reflux pendant 24 heures. Le mélange réactionnel est évaporé à sec sous pression réduite. Le résidu est chromatographié sur Silice (éluant : CH₂Cl₂/MeOH 98 :2), pour donner 7 g (Rendement : 87%) de fractions propres du 2-ethylhexyl aminoacetate sous forme d'une huile orangée et utilisée telle quelle dans l'étape suivante.

### Deuxième étape : Préparation du produit de l'exemple 3 :

A 0°C, en 15 minutes, on ajoute 3,4 ml de chlorure d'oxalyle (0,0384 mole) à de l'acide 3-phénylpropanoyle (4,81 g, 0,032 mole) dissout dans un mélange de 20 ml d'acétonitrile et 1 ml de DMF anhydres. On laisse sous agitation à température de labo pendant 3 heures. Les solvants sont évaporés sous pression réduite et l'huile jaune obtenue est reprise dans 20 ml d'acétonitrile. On refroidit à -5°C et goutte à goutte, on introduit le produit de l'étape précédente (6g, 0,032 mole) dissout dans 10 ml d'acétonitrile suivi de 16 ml de diisopropylethylamine. Le mélange réactionnel est agité pendant 17 heures. Il est évaporé à sec sous pression réduite. Le résidu est repris dans l'acétate d'éthyle et lavé avec une solution saturée en chlorure de sodium et deux fois à l'eau. La phase organique isolée est séchée sur sulfate de sodium. Après filtration et évaporation du solvant, l'huile orangée obtenue est chromatographiée sur Silice (éluant : Heptane/EtOAc 90 :10), pour donner 3,25 g (Rendement : 31%) de fractions propres du composé de l'exemple 3 sous forme d'une huile jaune pâle.

### EXEMPLE 4 : Préparation du N-(2-ethylhexyl)-3-phenyl-N-(2-phenylethyl)propanamide:

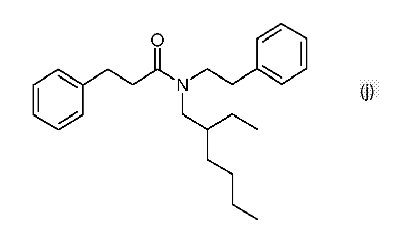

### Première étape : Préparation du N-(2-ethylhexyl)-N-(2-phenylethyl) :

A une suspension d'hydrure de sodium (à 50% dans l'huile, 1,49 g) dans 10 ml de THF anhydre, on ajoute en 15 minutes à 0 °C du 2-éthyl-1-hexylamine (4 g, 0,0309 mole) dissout dans 16 ml de THF anhydre puis on laisse 1 heure à température du labo. On ajoute ensuite en 15 minutes à température du labo du 2-bromoéthylbenzène (5,1 ml, 0,037 mole) dissout dans 60 ml de THF anhydre. On laisse le mélange réactionnel à température du labo pendant 19 heures puis 24 heures à 80°C. Après refroidissement , on ajoute 100 ml d'eau avec précaution , et extrait avec 3x150 ml de CH₂Cl₂. La phase organique est lavée avec une solution saturée en NaCl, séchée sur sulfate de sodium puis est évaporée à sec sous pression réduite. L'huile jaune obtenue est chromatographiée sur Silice (éluant : Heptane/EtOAc 50 :50), pour donner 3,4 g (Rendement : 47%) de fractions propres du N-(2-ethylhexyl)-N-(2-phenylethyl) sous forme d'une huile jaune et utilisée telle quelle dans l'étape suivante.

### Deuxième étape : Préparation du produit de l'exemple 4 :

A 0°C, en 10 minutes, on ajoute 1,5 ml de chlorure d'oxalyle (0,0145 mole) à de l'acide dihydrocinnamique (2,2 g, 0,0145 mole) dissout dans un mélange de 10 ml d'acétonitrile et 0,5 ml de DMF anhydres. On laisse sous agitation à température de labo pendant 3 heures. Les solvants sont évaporés sous pression réduite et l'huile orangée obtenue est reprise dans 20 ml d'acétonitrile. On refroidit à -5 °C et goutte à goutte, on introduit simultanément le produit de l'étape précédente (3,4 g, 0,0145 mole) dissout dans 10 ml d'acétonitrile et de 7,2 ml de diisopropylethylamine. Le mélange réactionnel est agité pendant 17 heures. Il est évaporé à sec sous pression réduite. Le résidu est repris dans l'acétate d'éthyle et lavé avec une solution saturée en chlorure de sodium et deux fois à l'eau. La phase organique isolée est séchée sur sulfate de sodium. Après filtration et évaporation du solvant, l'huile brune obtenue est chromatographiée sur Silice (éluant : Heptane/EtOAc 90 :10), pour donner 3,98 g (Rendement : 73%) de fractions propres du composé de l'exemple 4 sous forme d'une huile jaune pâle.

### EXEMPLE 5 : Préparation du 2-phényléthyl 3,4-diméthylbenzoate:

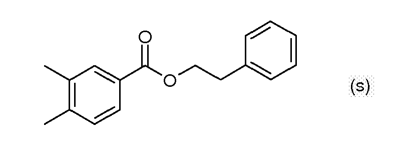

On chauffe à 80°C pendant 20 heures un mélange d'acide 3,4-diméthyl benzoïque (16,53 g, 0,11 mole), de 2-phényl éthanol (15,8 ml, 0,132 mole) et d'acide sulfurique à 98% (0,6 ml, 0,011 mole) dans 170 ml de toluène. Le mélange réactionnel brun est lavé à l'eau. Après séparation de la phase organique, le solvant est évaporé sous pression réduite. Le résidu est repris dans un mélange Heptane/AcOEt 97 : 3 et est filtré sur lit de silice. Après isolation des fractions propres et après évaporation du solvant, on obtient 20,2 g (Rendement : 72%) du composé de l'exemple 1 sous forme d'une huile jaune pâle.

### EXEMPLE 6 : Préparation du bis(2-phenylethyl) pyridine-2,4-dicarboxylate :

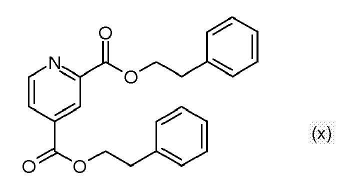

On chauffe à 80°C pendant 15 heures le mélange de 2,4-pyridine dicarboxylic acid (6 g, 0,0359 mole), de 2-phényléthanol (10,16 ml, 0,085 mole) et de 0,6 ml d'acide sulfurique concentré dissout dans 60 ml de toluène. Après évaporation des solvants sous pression réduite, l'huile jaune visqueuse obtenue est solubilisée dans le dichlorométhane et est purifiée sur lit de silice. Après évaporation du solvant, on obtient 5,8g (Rendement : 43%) des fractions propres du produit de l'exemple 2 sous forme d'un solide blanc.

### EXEMPLES DE FORMULATION 7 à 9

On a réalisé les compositions suivantes puis évalué pour chacune d'entre elles la photostabilité du 4-Tertiobutyl-4'-Methoxy-Dibenzoylmethane

| Compositions | Ex7 comparatif | EX8 | EX9 |
|---|---|---|---|
| Poly Dimethylsiloxane | 0,5 | 0,5 | 0,5 |
| Conservateur | 1 | 1 | 1 |
| Acide Stéarique | 1,5 | 1,5 | 1,5 |
| Mélange mono-stéarate de glycéryle / stéarate de PEG (100 OE) (ARLACEL P165-UNIQUEMA) | 1 | 1 | 1 |
| Eau désionisée | 73,35 | 73,35 | 73,35 |
| Chélatant | 0,1 | 0,1 | 0,1 |
| N-Lauroyl sarcosinate d'isopropyle (ELDEW SL-205 - AJINOMOTO) (composé amide) | 10 | 9 | 9 |
| Amide de mono ou diarylalkyle (composé I) | | 1 | |
| Amide de mono ou diarylalkyle (composé a) | | | 1 |
| 4-Tertiobutyl-4'-Methoxy-Dibenzoylmethane (PARSOL 1789- DSM NUTRITIONAL PRODUCTS) | 2 | 2 | 2 |
| Glycérol | 5 | 5 | 5 |
| Gomme de Xanthane | 0,2 | 0,2 | 0,2 |
| Potassium Cetyl Phosphate (AMPHISOL K-DSM NUTRITIONAL PRODUCTS) | 1 | 1 | 1 |
| Iso-Hexadecane | 1 | 1 | 1 |
| Copolymère acide acrylique/méthacrylate de stéaryle (PEMULEN TR 1- NOVEON) | 0,2 | 0,2 | 0,2 |
| Alcool cétylique | 0,5 | 0,5 | 0,5 |
| Triethanolamine | 0,65 | 0,65 | 0,65 |
| Mélange de cétylstéaryl glucoside et d'alcools cétylique, stéarylique (MONTANOV 68 -SEPPIC) | 2 | 2 | 2 |

### Méthode de mesure

Pour chaque formule, on a préparé 3 échantillons tests et 3 échantillons témoins. On dépose, à la spatule, 2 mg/cm2 de formule sur des plaques de polyméthacrylate de méthyle.

Les plaques tests sont exposées 38 mn au SUN TEST HERAUS muni d'une lampe Xénon ayant un flux UV-A de 9.5 10⁻³ W/cm² et un flux UV-B de 5.43.10⁻⁴ W/cm². Les plaques témoins sont conservées pendant le même temps et à la même température (38-40°C) à l'obscurité.

A l'issue de ce temps, on procède à l'extraction des filtres en immergeant chaque plaque dans 50 g méthanol et en les soumettant aux ultrasons pendant 15 mn pour assurer une bonne extraction. Les solutions obtenues sont analysées par HPLC et spectrophotométrie UV.

Pour chaque formule testée, le taux de 4-tertiobutyl-4'-méthoxy-dibenzoylmethane résiduel après exposition est donné dans le rapport de sa densité optique (DO) dans l'échantillon exposé à sa densité optique (DO) non exposé. On se place au maximum d'absorption correspondant au butyl-méthoxy-dibenzoylméthane : λmax = 358 nm.

Les résultats obtenus sont résumés dans le tableau 1 suivant :

| Compositions | % résiduel de dibenzoylméthane après exposition 1 heure UVA |
|---|---|
| Exemple 7 (comparatif) | 74% |
| Exemple 8 | 80 % |
| Exemple 9 | 79 % |

On observe une amélioration de la photostabilité du dibenzoylméthane en présence d'un composé amide d'arylalkyle de formule (I) (composé a) ou (II) (composé I) par rapport au N-Lauroyl sarcosinate d'isopropyle (ELDEW SL-205 - AJINOMOTO) (composé amide de l'art antérieur).

### EXEMPLES DE FORMULATION 10 à 12 :

On a réalisé les compositions suivantes puis évalué pour chacune d'entre elles la photostabilité du 4-Tertiobutyl-4'-Methoxy-Dibenzoylmethane selon la méthode indiquée dans les exemples 7 à 9 précédents.

| Compositions | EX10 (comparatif) | EX11 | EX12 |
|---|---|---|---|
| Poly Dimethylsiloxane | 0,5 | 0,5 | 0,5 |
| Conservateurs | 1 | 1 | 1 |
| Acide Stéarique | 1,5 | 1,5 | 1,5 |
| Mélange mono-stéarate de glycéryle / stéarate de PEG (100 OE) (ARLACEL P165-UNIQUEMA) | 1 | 1 | 1 |
| Eau désionisée | 73,35 | 73,35 | 73,35 |
| Sequestrant | 0,1 | 0,1 | 0,1 |
| Benzoate de 2-éthylphenyle (X-TEND 226 ® - ISP) | 20 | | |
| Ester d'arylalkyle (composé s) | | 20 | |
| Ester d'arylalkyle (composé v) | | | 20 |
| 4-Tertiobutyl-4'-Methoxy-Dibenzoylmethane (PARSOL 1789 ® DSM NUTRITIONAL PRODUCTS) | 2 | 2 | 2 |
| Glycérol | 5 | 5 | 5 |
| Gomme de Xanthane | 0,2 | 0,2 | 0,2 |
| Potassium Cetyl Phosphate (AMPHISOL K-DSM NUTRITIONAL PRODUCTS) | 1 | 1 | 1 |
| Iso-Hexadecane | 1 | 1 | 1 |
| Copolymère acide acrylique/méthacrylate de stéaryle (PEMULEN TR 1- NOVEON) | 0,2 | 0,2 | 0,2 |
| Alcool cétylique | 0,5 | 0,5 | 0,5 |
| Triethanolamine | 0,65 | 0,65 | 0,65 |
| Mélange de cétylstéaryl glucoside et d'alcools cétylique, stéarylique (MONTANOV 68 -SEPPIC) | 2 | 2 | 2 |

Les résultats obtenus sont résumés dans le tableau suivant :

| Compositions | % résiduel de di-benzoylméthane après exposition 1 heure UVA |
|---|---|
| Formule 10 (comparatif) | 52 % |
| Formule 11 | 74% |
| Formule 12 | 65% |

On observe une amélioration sensible de la photostabilité du dibenzoylméthane en présence d'ester d'arylalkyle (composés (s) et (v)) par rapport au Benzoate de 2-éthylphenyle (X-TEND 226 ® - ISP)

## Revendications

1. Composition contenant dans un milieu cosmétiquement acceptable au moins un filtre UV-A du type dérivé de dibenzoylméthane et au moins un composé amide ou ester d'arylalkyle de formule (I), (II) ou (III) suivante : 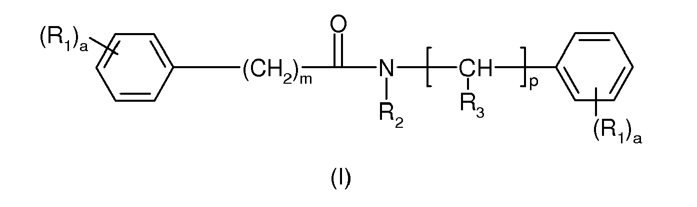 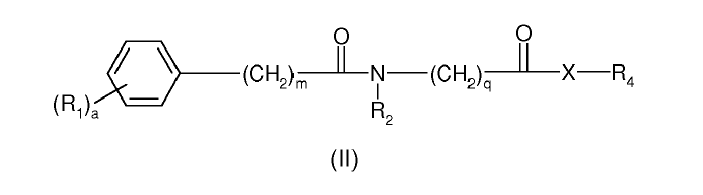 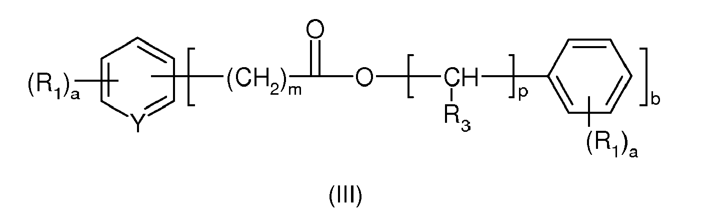 dans lesquelles
- R₁ est un radical alkyle en C₁-C₄, linéaire ou ramifié,
- a est 0 - 2,
- m est 0 - 4,
- b est 1 ou 2,
- R₂ est H, un alkyle en C₁-C₂₀, linéaire ou ramifié, ou le groupement acétyle,
- R₃ de manière indépendante est H ou méthyle,
- p est 0 - 4,
- q est 0 - 3,
- X est O, NH ou NR₅,
- Y est CH ou N,
- R₄ et R₅ sont des radicaux alkyles en C₁-C₂₀, linéaires ou ramifiés,
avec la condition suivante pour la formule(III) : si Y = CH, R₃ = H, m = 0, p = 2, alors a est différent de 0 et R₁ différent de méthyle.

2. Composition selon la revendication 1, où le composé de formule (I) est choisi parmi les produits (a) à (k) suivants :

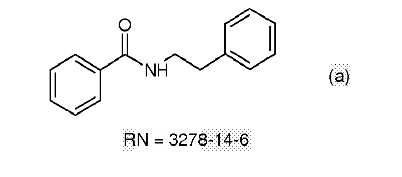

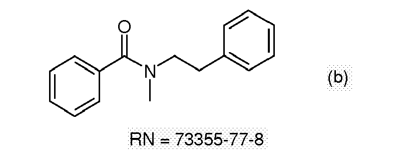

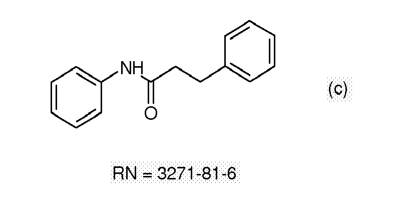

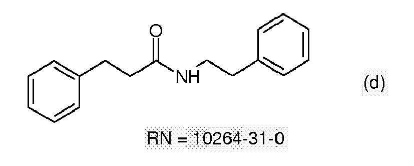

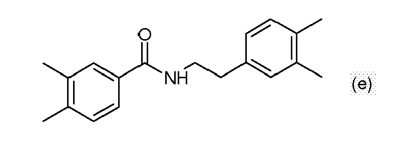

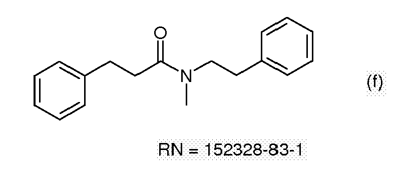

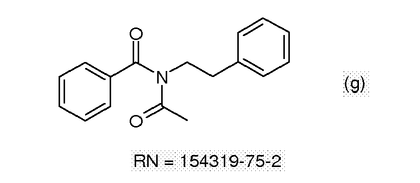

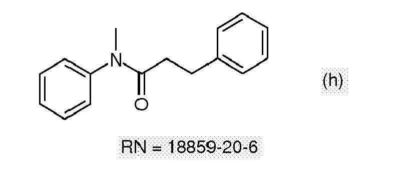

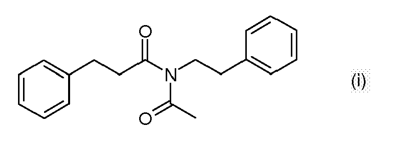

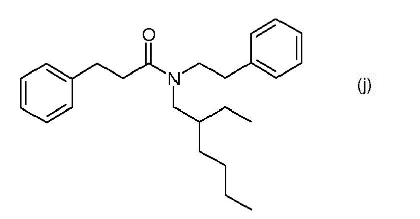

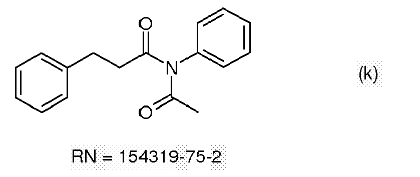

3. Composition selon la revendication 1, où le composé de formule (II) est choisi parmi les produits (I) à (r) suivants :

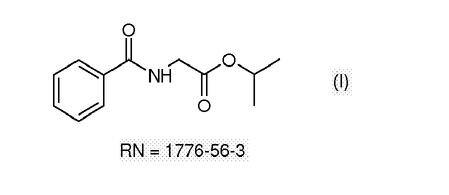

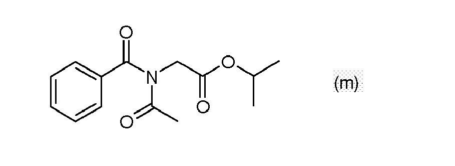

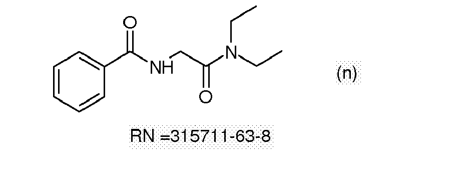

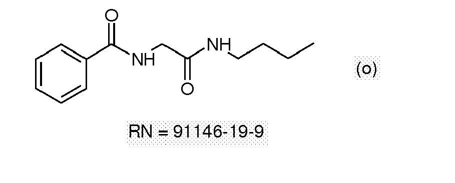

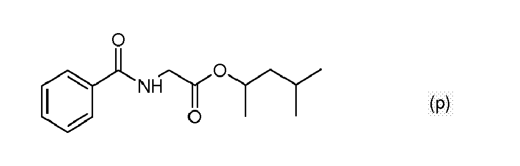

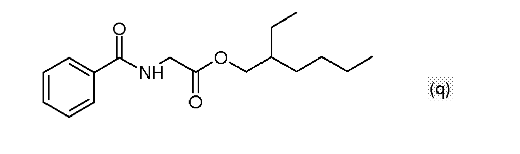

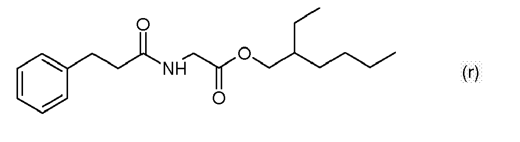

4. Composition selon la revendication 1, où le composé de formule (III) est choisi parmi les produits (s) à (f) suivants :

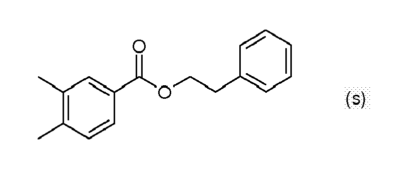

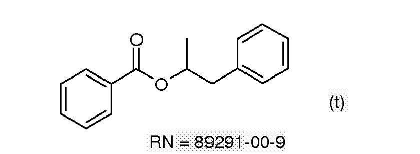

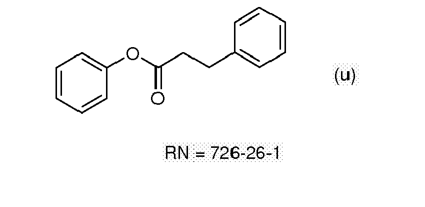

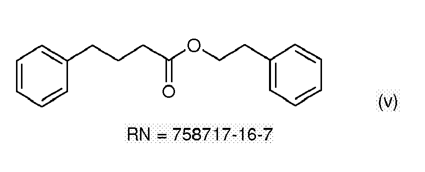

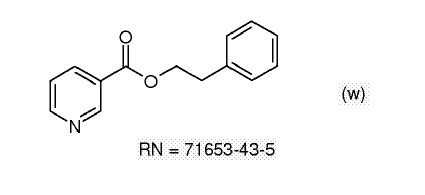

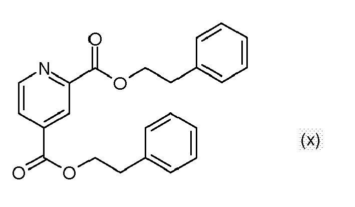

5. Composition selon l'une quelconque des revendications précédentes, où le ou les composés amides ou esters d'arylalkyle de formule (I), (II) ou (III) sont présents dans des proportions allant de 0,01 % à 20 % en poids, de préférence de 0,1 % à 10 % en poids, par rapport au poids total de la composition.

6. Composition selon l'une quelconque des revendications précédentes, où le dérivé de dibenzoylméthane est choisi parmi :
- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5-diméthyldibenzoylméthane,
- le 4,4'-diisopropyldibenzoylméthane,
- le 4,4'-diméthoxydibenzoylméthane,
- le 4-tert.-butyl-4'-méthoxydibenzoylméthane,
- le 2-méthyl-5-isopropyl-4'-méthoxydibenzoylméthane,
- le 2-méthyl-5-tert-butyl-4'-méthxydibenzoylméthane,
- le 2,4-diméthyl-4'-méthoxydibenzoylméthane,
- le 2,6-diméthyl-4-tert-butyl-4'-méthoxydibenzoylméthane.

7. Composition selon la revendication 5, où le dérivé de dibenzoylméthane est le 4-isopropyl-dibenzoylméthane répondant à la formule suivante :

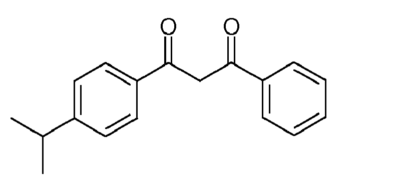

8. Composition selon la revendication 5, où le dérivé de dibenzoylméthane est le 4-(ter.-butyl) 4'-méthoxy dibenzoylméthane ou Butyl Methoxy Dibenzoylmethane de formule suivante :

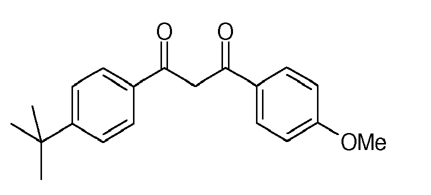

9. Composition selon l'une quelconque des revendications précédentes, où le ou les dérivés de dibenzoylméthane sont présents à des teneurs qui varient de 0,01 à 10% en poids et plus préférentiellement de 0,1 à 6% en poids par rapport au poids total de la composition.

10. Composition selon l'une quelconque des revendications précédentes, **caractérisée par le fait qu'**elle contient en plus d'autres filtres UV organiques ou inorganiques actifs dans l'UV-A et/ou l'UV-B hydrosolubles ou liposolubles ou bien insolubles dans les solvants cosmétiques couramment utilisés.

11. Composition selon la revendication 10, où les filtres UV organiques complémentaires sont choisis parmi les anthranilates ; les dérivés cinnamiques ; les dérivés salicyliques, les dérivés du camphre ; les dérivés de la benzophénone ; les dérivés de β,β-diphénylacrylate ; les dérivés de triazine ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de méthylène bis-(hydroxyphényl benzotriazole) ; les dérivés de benzoxazole ; les polymères filtres et silicones filtres ; les dimères dérivés d'α-alkylstyrène ; les 4,4-diarylbutadiènes et leurs mélanges.

12. Composition selon la revendication 10, **caractérisée par le fait que** le ou les filtres UV organiques sont choisis parmi les composés suivants :
Ethylhexyl Methoxycinnamate
Homosalate
Ethylhexyl Salicylate,
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
Methylène bis-Benzotriazolyl Tetramethylbutylphénol
Ethylhexyl triazone,
Bis-Ethylhexyloxyphenol Methoxyphenyl Triazine
Diethylhexyl Butamido Triazone,
2,4,6-tris(4'-amino benzalmalonate de dinéopentyle)-s-triazine
la 2,4,6-tris-(4'-amino benzalmalonate de diisobutyle)-s-triazine.
la 2,4,6-tris(biphenyl-4-yl-1,3,5-triazine)
la 2,4,6-tris(terphenyl)-1,3,5-triazine
Drometrizole Trisiloxane
Drometrizole Trisiloxane
Polysilicone-15
Di-néopentyl 4'-méthoxybenzalmalonate
1,1-dicarboxy (2,2'-diméthyl-propyl)-4,4-diphénylbutadiène
2,4-bis-[5-1 (diméthylpropyl)benzoxazol-2-yl-(4-phenyl)-imino]-6-(2-ethylhexyl)-imino-1,3,5-triazine
et leurs mélanges.

13. Composition selon la revendication 10, **caractérisée par le fait que** les filtres UV inorganiques complémentaires sont des pigments d'oxydes métalliques, traités ou non.

14. Composition selon la revendication 13, **caractérisée par le fait que** lesdits pigments sont choisis parmi les oxydes de titane, de zinc, de fer, de zirconium, de cérium et leurs mélanges, traités ou non.

15. Composition selon l'une quelconque des revendications 1 à 14, **caractérisée par le fait qu'**elle comprend en outre au moins un agent de bronzage et/ou de brunissage artificiel de la peau.

16. Composition selon l'une quelconque des revendications 1 à 14, **caractérisée par le fait qu'**elle comprend en outre au moins un adjuvant choisi parmi les corps gras, les solvants organiques, les épaississants ioniques ou non ioniques, hydrophiles ou lipophiles, les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, des actifs, les charges, les polymères, les propulseurs, les agents alcalinisants ou acidifiants

17. Composition selon l'une quelconque des revendications 1 à 16, **caractérisée par le fait qu'**elle se présente sous la forme d'une émulsion huile-dans-eau ou eau-dans huile.

18. Utilisation d'une composition telle que définie dans l'une quelconque des revendications 1 à 17 pour la fabrication de produits pour le traitement cosmétique de la peau, des lèvres, des ongles, des cheveux, des cils, sourcils et/ou du cuir chevelu

19. Utilisation d'une composition telle que définie dans l'une quelconque des revendications 1 à 17 pour la fabrication de produits de soin de la peau, des lèvres, des ongles, des cheveux et/ou du cuir chevelu.

20. Utilisation d'une composition telles que définie dans l'une quelconque des revendications 1 à 17 pour la fabrication de produits de maquillage.

21. Procédé pour améliorer la stabilité chimique vis-à-vis du rayonnement UV d'au moins un filtre UV-A du type dérivé du dibenzoylméthane tel que défini dans l'une quelconque des revendications précédentes, **caractérisé par le fait qu'**on associe audit dérivé de dibenzoylméthane au moins un composé amide ou ester d'arylalkyle de formule (I) (II) ou (III) tel que défini dans les revendications précédentes.

22. Utilisation d'au moins un composé amide ou ester d'arylalkyle de formule (I), (II) ou (III) tel que défini dans les revendications précédentes dans une composition comprenant dans un support cosmétiquement acceptable, au moins un dérivé du dibenzoylméthane tel que défini dans l'une quelconque des revendications précédentes dans le but de d'améliorer l'efficacité de ladite composition vis-à-vis des rayons UV-A.
